# EUROPEAN PATENT SPECIFICATION

(11) **EP 1 676 582 B1**
(45) Date of publication and mention of the grant of the patent: **24.12.2014**
(21) Application number: 03818872.8
(22) Date of filing: 31.10.2003
(51) Int. Cl.: A61K 33/38, A61P 31/12, A61K 47/02, A61K 9/00, A61K 47/26

(54) **ANTI-CORONAVIRUS AGENT**
ANTI-CORONAVIRUS-MITTEL
AGENT LUTTANT CONTRE LE CORONAVIRUS

(30) Priority: 16.10.2003 JP 2003356935
(43) Date of publication of application: 05.07.2006
(73) Proprietor: TOAGOSEI CO., LTD., Tokyo 105-8419 (JP)
(72) Inventor: SUGIURA, Koji, c/o Toagosei Co., Ltd., Nagoya-shi, Aichi 455-0027 (JP); ONO, Yasuharu, c/o Toagosei Co., Ltd., Nagoya-shi, Aichi 455-0027 (JP)
(74) Representative: Kotitschke & Heurung Partnerschaft mbB
(86) International application number: PCT/JP2003/014008
(87) International publication number: WO 2005/037296

(56) References cited:
- WO-A-00/61367
- DE-A1- 4 339 374
- JP-A- 5 105 609
- JP-A- 6 263 916
- US-A- 3 930 000
- KATO ET AL: "Dentifrices cotaining bactericidal metal phosphates" STN CHEMICAL ABSTRAC, 12 July 1993 (1993-07-12), XP002124336
- RENTZ DO COMM CNMO E. J.: 'Viral pathogens and severe acute respiratory syndrome: oligodynamic Ag+ for direct immune intervention' JOURNAL OF NUTRITIONAL & ENVIRONMENTAL MEDICINE vol. 13, no. 2, 2003, pages 109 - 118, XP009022135

## Description

### Technical Field

The present invention relates to an anti-coronaviral agent that is effective in treating coronaviruses and can be mixed with plastics, paints and the like. It also relates to an anti-coronaviral product made from fibers, resins and others that contain the anti-coronaviral agent.

### Background Art

In recent years, various infectious diseases have become social problems. In particular, there is no vaccine or no causal treatment yet against Severe Acute Respiratory Syndrome (SARS) which is caused by SARS coronavirus and was epidemic in Southeast Asia, and therefore it is very important to prevent the infection. There have been reports that SARS infections could be spread not only through direct contact with or by droplet infection from infected persons but also through indirect contact via door knobs, clothing and other articles which infected persons have touched. According to the first data collected by the network of WHO collaborating centers on stability and resistance of SARS coronavirus, SARS coronavirus can survive for a few days outside the body in certain conditions. Therefore, for the purpose of preventing the infection, it is considered effective to keep clean the articles which would be frequently touched by infected persons by means of disinfection and the like.

It has been reported that ethanol, sodium hypochlorite, iodophor, peracetic acid, formaldehyde, glutaraldehyde and ethylene However, these disinfectants are effective only temporarily and thus may be useful only when a coronavirus is apparently placed there. Since these disinfectants are not expected to have sustained efficacy, they are problematic in that they are not always able to maintain various articles in clean condition.

On the other hand, silver-based inorganic anti-microbial agents have been known as anti-microbial agents that have sustained efficacy against bacteria. The silver-based inorganic anti-microbial agents are anti-microbial agents which comprise silver carried on an inorganic compound. They can be processed into various useful product forms because of their high heat resistance. As examples thereof, active charcoal on which silver and/or silver compounds are carried (for example, refer to JP Patent Publication (Kokai) No. 49-61950), soluble glass containing silver (for example, refer to JP Patent Publication (Kokai) No. 63-307807), zeolite containing silver, copper and/or zinc (for example, refer to JP Patent Publication (Kokai) No. 60-181002), zirconium phosphate containing silver (for example, refer to JP Patent Publication (Kokai) No. 3-83905) may be mentioned. However, it has not been reported that these silver-based inorganic anti-microbial agents are effective against coronaviruses such as SARS coronavirus.

It has been reported that silver nitrate is effective against HSV (herpes simplex virus) -1 and -2, but is not effective against vacciniavirus, Adenovirus, VSV, Poliovirus or HVJ (for example, refer to R. B. Thurman, C. P. Gerba, "The Molecular Mechanisms of Copper and Silver ion Disinfection of Bacteria and Viruses", Critical Reviews in Environmental Control, 1989, Vol. 18, No. 4, p.295-315).

DE 4339374 A1 discloses a composition comprising silver ion carriers which is used for the control of bacteria, fungi and algae, respectively. However, DE 4339374 A1 does not disclose the use of an agent comprising a silver ion carrier for inactivating a coronavirus.

Eric J. RENTZ DO COMN CNMO, "Viral Pathogens and Severe Acute Respiratory Syndrome: Oligodynamic Ag+ for Direct Immune Intervention", Journal of Nutritional & Environmental Medicine, Vol. 13, No. 2, p.109-118, discloses the use of charged silver particles, e.g. nebulized Ag⁺, as antibacterial and antiviral drugs. Said document suggests that nebulized oligodynamic Ag⁺ is likely to have an effect against SARS-related coronavirus. Moreover, said document teaches that depending upon the type of silver formulation used, outcomes vary widely, and that e.g. silver nitrate, silver sulfadiazine and electrolytically produced Ag⁺ all had different antiviral properties. Furthermore, said document discloses that oligodynamic Ag⁺ is an effective biocide at concentrations ranging between 0.0000001 and 0.00006% (which is equivalent to 9.2 ppb to 5.5 ppm), and that oligodynamic Ag⁺ was a highly effective biocide in concentrations of 50 ppm over 4 hours or less, and in concentrations of 250 ppb over 2 hours or less.

US 5,296,238 discloses a microbicide phosphate represented by the general formula Mₐ¹A_{b}M_{c}²(PO₄)_{d}·nH₂O, wherein M¹ is silver, M² is zirconium or titanium, A represents at least one selected from the group consisting of hydrogen ion, alkali metal ions, and ammonium ion, n represents a number which satisfies 0≤n≤6, a and b each represents a positive number and satisfies the equation 1a+mb=I, where I is valence of M¹ and m is valence of A, and c is 2 and d is 3. US 5,296,238 discloses a very low Ag⁺ elution amount of compounds of the above formula of 0.1 ppm or lower.

### Disclosure of the Invention

An object of the present invention is to provide an anti-viral agent which is effective against a coronavirus and can be expected to have sustained efficacy when it is processed into various products.

The present inventors have performed intensive researches in order to solve the above problem and have found that the problem can be solved by using a silver ion as an active ingredient and having the silver ion stably held by a carrier. Thus, the present inventors have completed the present invention.

The present invention relates to the use of an anti-viral agent as defined in claim 1 effective against a coronavirus, which is characterized in that it comprises a carrier which holds a silver ion stably. The present invention discloses a product containing an anti-coronaviral agent, which is useful for inactivating a coronavirus, and also discloses a method for inactivating a coronavirus using the silver ion carrier according to the present invention.

The present invention has been completed based on the above findings and is shown below.
1. Use of an anti-coronaviral agent which comprises a silver ion carrier according to present claims 1 to 6, wherein the aforementioned silver ion carrier is a compound represented by the following formula (1):

   AgₐA_{b}M2_{c}(PO₄)_{d} · nH₂O (1)

   wherein A is at least one m-valent ion (m is a positive integer) selected from an alkali metal ion, an alkali earth metal ion, an ammonium ion and a hydrogen ion; M2 is an ion of a tetravalent metal selected from zirconium and titanium; n is a number satisfying the condition of 0 ≤ n ≤ 6; a and b are positive numbers satisfying a condition of a + mb = 1 or a + mb = 2; and c = 2 and d = 3 when a + mb = 1, and c = 1 and d = 2 when a + mb = 2.

### Best Mode for Carrying Out the Invention

The present invention will be described below.

Cornavirus in the present invention means a single stranded (+) RNA virus of the Coronavirus family including the genus Coronavirus and genus Torovirus. Also included are infectious bronchitis virus (IBV), feline infectious peritonitis virus (FIP), canine coronavirus (CCV), swine transmissible gastroenteritis virus (TGEV), equine torovirus (EqTV) and the like. This name was given because the viruses have, on the surface of the envelope thereof, projections which show an appearance resembling the corona of the Sun. The SARS epidemic in Southeast Asia in 2003 was caused by a new species of this coronavirus.

Silver ion which is an active ingredient of the anti-viral agent used according to the present invention must be stably held in its carrier. The sentence "stably held" means that the silver ion is maintained to be in an ionic state regardless of heat (for example, at a temperature of 200°C or below) or water (for example, at a humidity of 10 to 100%) but is not converted to a non-ionic state exemplified by a metallic or oxidized state.

The carrier which holds a silver ion stably according to the present invention includes compounds represented by the general formula (1), such as zirconium phosphate.

The compounds represented by the above described formula (1) may be obtained from a compound represented by the general formula (2).

A'ₓZr₂(PO₄)₃ (2)

In the formula (2), A' is at least one metal ion selected from an alkali metal ion, an alkali earth metal ion or an ammonium ion, x is 1 when A' is monovalent and 1/2 when A is divalent. By immersing this compound in an aqueous solution containing an appropriate concentration of a silver ion at room temperature to 100°C, the compounds represented by the formula (1) can be obtained.

Preferred examples represented by the general formula (1) include the followings:
Ag_{0.01}Na_{1.99}Zr(PO₄)₂
Ag_{0.1}(NH₄)_{1.9}Ti(PO₄)₂·4H₂O
Ag_{0.005}Li_{0.995}Zr₂(PO₄)₃
Ag_{0.01}(NH₄)_{0.99}Zr₂(PO₄)₃
Ag_{0.05}Na_{0.95}Zr₂(PO₄)₃
Ag_{0.2}K_{0.8}Ti₂(PO₄)₃
Ag_{0.1}H_{0.9}Zr₂(PO₄)₃
Ag_{0.4}H_{0.15}Na_{0.45}Zr₂(PO₄)₃
Ag_{0.6}H_{0.1}Na_{0.3}Zr₂(PO₄)₃

In the present invention, the silver ion is contained in the silver ion carrier at a concentration of 0.05 to 15% by mass and especially preferably 0.1 to 5% by mass. When the silver ion is contained in the silver ion carrier at a concentration of 0.01% by mass or less, anti-viral effect against a coronavirus may not be obtained; when the silver ion is contained therein at a concentration of 20% by mass or more, products containing the silver ion carrier become colored or economically disadvantageous, and thus these cases are not preferable.

The present anti-coronaviral agent preferably elutes silver ions to water in an amount of 0.5 µg/liter/day or more (per 10 g of the silver ion carrier, for example), and more preferably 5 µg/liter/day or more from the viewpoint of sustainability of the efficacy. However, too much elution of silver ions is not preferable because silver ions are eluted out beyond necessity for the effect and also because products containing the silver ion carrier are colored.

Further, the particle diameter of the carrier is preferably 0.1 to 15 µm and more preferably 0.5 to 10 µm, because the efficacy is more efficiently exhibited within the range of diameter.

The anti-coronaviral agent used according to the present invention can be obtained by mixing and stirring a solution of a silver compound with a carrier such as zirconium phosphate represented by the general formula (1). The silver compound which may be used includes silver nitrate, silver sulfate, silver perchlorate, silver acetate, silver diammine nitrate, silver diammine sulfate and the like. The mixing may be carried out at 10 to 80°C, preferably 40 to 60°C, for 1 to 50 hours, preferably 5 to 24 hours, by batch method or continuous method. After the mixing, the carrier is washed with water and dried at 60 to 170°C. Water soluble glass may also be prepared by allowing it to include silver oxide or the silver compound described above.

Since the anti-coronaviral agent used according to the present invention is powder, it may be used as it is or after being processed. For example, it may be used in suspension or processed into shaped articles such as particles, paper-like materials, pellets, sheets, films and the like. Also, it may be in a form of porous materials or fibers. Further, it can be processed into paints, cloth, non woven cloth, foam sheets, paper, plastics and inorganic boards.

Production examples of products using the anti-coronaviral agent of the present invention will be described below.

### Production examples using silver zirconium phosphate-based carriers

A silver zirconium phosphate-based carrier that contains 3.7% by mass of silver is mixed with polyester resin for fiber in a proportion of 1% by mass, and by a standard melt spinning method, an anti-coronavirus polyester multifilament composed of 75 of 2 denier filaments can be obtained. From the filament, cloth and paper can be produced.

A silver zirconium phosphate-based carrier that contains 3.7% by mass of silver is mixed with polypropylene resin in a proportion of 10% by mass to produce a master batch containing 10% by mass of the carrier. After mixing this master batch with polypropylene resin in a proportion of 10% by mass, an anti-coronavirus polypropylene molded product can be obtained by a standard injection molding method. The molded product can be used as an anti-coronaviral product.

A silver zirconium phosphate-based carrier that contains 3.7% by mass of silver is mixed with UV curing acryl paint in a proportion of 1% by mass to produce an anti-coronavirus UV paint. An anti-coronavirus SUS board can be obtained by painting this anti-coronavirus paint on a SUS board by a standard method. This anti-coronavirus paint can be used for painting walls, ceilings, floors and the like.

### Production examples using silver zeolite Y based carrier (not according to the present invention)

A silver zeolite Y based carrier that contains 2.0% by mass of silver is mixed with polyamide resin for fiber in a proportion of 1% by mass, and by a standard melt spinning method, an anti-coronavirus polyamide multifilament composed of 75 of 2 denier filaments can be obtained. From the filament, cloth and paper can be produced.

A silver zeolite A based carrier that contains 1.1% by mass of silver is mixed with ABS resin in a proportion of 10% by mass to produce a master batch containing 10% by mass of the carrier. After mixing this master batch with ABS resin in a proportion of 10% by mass, an anti-coronavirus ABS molded product can be obtained by a standard injection molding method. The molded product can be used as an anti-coronaviral product.

### Production examples using silver glass based carrier (not according to the present invention)

A silver glass based carrier that contains 1.0% by mass of silver is mixed with polyester resin for fiber in a proportion of 0.5% by mass, and by a standard melt spinning method, an anti-coronavirus 30 denier polyester monofilament can be obtained. From the filament, cloth and paper can be produced.

A silver glass based carrier that contains 1.0% by mass of silver is mixed with polystyrene resin in a proportion of 10% by mass to produce a master batch containing 10% by mass of the carrier. After mixing this master batch with polystyrene resin in a proportion of 10% by mass, an anti-coronavirus polystyrene molded product can be obtained by a standard injection molding method. The molded product can be used as an anti-coronaviral product.

### Production examples using silver carrying silica gel based carrier (not according to the present invention)

A silver silica gel based carrier that contains 1.1% by mass of silver is mixed with polypropylene resin for fiber in a proportion of 1% by mass, and by a standard melt spinning method, an anti-coronavirus 30 denier polypropylene monofilament can be obtained. From the filament, cloth and paper can be produced.

A silver silica gel based carrier that contains 1.1% by mass of silver is mixed with polyethylene resin in a proportion of 10% by mass to produce a master batch containing 10% by mass of the carrier. After mixing this master batch with polyethylene resin in a proportion of 10% by mass, an anti-coronavirus polyethylene molded product can be obtained by a standard injection molding method. The molded product can be used as an anti-coronaviral product.

The products of the present invention can be used in various fields and places where detoxification of coronavirus is desired. For example, they can be used in medical field, cosmetic field, apparel field, bedding field, household field, building material field, seacraft field, water treatment field and the like. In the medical field, they can be used as operation tools, adhesive tapes, containers for medical wastes, linens and the like. Particular examples of usage of the anti-coronaviral agent of the present invention include doors, floors, walls and the like, as well as medicine wear, gloves, shoe covers, masks, curtains, sheets, towels, dishcloths and the like. It may be integrated as filters into air cleaners, air conditioners and the like.

### EXAMPLES

The present invention will be further described by way of the following examples.

### Example 1

A virus inactivation test of a silver zirconium phosphate (Novaron AG300 (trade name) manufactured by Toagosei Co. Ltd.) was carried out against SARS coronavirus (SARS-COV-P11 and SARS-COV-P8) isolated from SARS patients.

### Monolayer plates of African green monkey kidney cell line VERO E6 (VERO E6 monolayer plates)

4 x 10⁵ cells/ml of VERO E6 cells were inoculated to a 96-well plate and cultured in an incubator at 37°C in 5% CO₂ for 24 hours to form a cell monolayer.

### Toxicity of silver zirconium phosphate against African green monkey kidney cell line VERO E6

Toxicity of silver zirconium phosphate against VERO E6 cells was measured. In particular, 6000 to 187.5 µg/ml solutions of silver zirconium phosphate were prepared in Eagles's cell culture medium and were added to VERO E6 monolayer plates, and cells were cultured at 37°C in 5% CO₂ for 5 to 7 days. Toxicity was monitored by observing cells under a microscope every day. The results indicated that the toxicity of silver zirconium phosphate was as follows.
TD₀ = 1500 µg/ml
TD₅₀ = 3000 µg/ml

### Toxicity test of SARS coronavirus

Toxicity of SARS-COV-P11 and SARS-COV-P8 against VERO E6 cells was determined according to cytopathic effect (CPE) method to determine TCID₅₀.

### Inactivation of SARS coronavirus by silver zirconium phosphate

To a culture containing SARS coronavirus in an amount of 100 TCID₅₀ determined as described above, silver zirconium phosphate was added so that it was contained therein at a concentration of 1500 µg/ml. And, the mixture was stirred for 4 and 6 hours. The resultant cultures were serially diluted two folds to prepare the test solutions so that the concentrations of silver zirconium phosphate were 1500 to 23.4 µg/ml equivalent. Each of the test solutions was added to wells of VERO E6 monolayer plates (4 wells were used for each concentration) and cultured at 37°C in 5% CO₂ for 5 to 7 days. During the culturing period, viral CPE was observed under a microscope everyday and the test was terminated when the CPE of the untreated control virus was 51% or above. The 100% inactivation concentration, 50% inactivation concentration and 25% inactivation concentrations of silver zirconium phosphate against SARS coronavirus were obtained and the results are shown in Table 1 (µg/ml).

**Table 1**

| | 100% Inactivation | 50% Inactivation | 25% Inactivation |
|---|---|---|---|
| 4 hours | 93.75, 93.75 | 46.8, 46.8 | 23.4, 23.4 |
| 6 hours | 93.75, 93.75 | 46.8, 46.8 | 23.4, 23.4 |

The results demonstrate that silver zirconium phosphate was effective against SARS coronavirus at the concentration of 23.4 µg/ml or above.

### Production Example

A silver zirconium phosphate-based carrier that contained 3.7% by mass of silver was mixed with polyester resin for fiber in a proportion of 1% by mass, and by a standard melt spinning method, an anti-coronavirus polyester multifilament composed of 75 of 2 denier filaments were obtained.

A silver zirconium phosphate-based carrier that contained 3.7% by mass of silver was mixed with polypropylene resin in a proportion of 10% by mass to produce a master batch containing 10% by mass of the carrier. After mixing this master batch with polypropylene resin in a proportion of 10 mass%, an anti-coronavirus polypropylene molded product was obtained by a standard injection molding method.

A silver zirconium phosphate-based carrier that contained 3.7% by mass of silver was mixed with a UV curing acryl paint in a proportion of 1% by mass to produce an anti-coronavirus UV paint. An anti-coronavirus SUS board was obtained by painting this anti-coronavirus paint on a SUS board by a standard method.

### Industrial Applicability

The anti-coronaviral agent using the silver ion carrier according to the present invention is effective against coronaviruses, durable and heat-resistant. For this reason, it can be processed with plastics and the like, and thus utilized as anti-coronaviral products in various forms. Since coronaviruses already present in or newly entering a room and the like can be inactivated by using the present silver ion carrier, infection can be prevented.

## Claims

1. Use of an agent comprising a silver ion carrier selected from compounds represented by the following formula (1) containing silver ions at a concentration of 0.05 to 15 % by mass for inactivating a coronavirus:
AgₐA_{b}M2_{c}(PO₄)_{d} · nH₂O (1)
wherein A is at least one m-valent ion (m is a positive integer) selected from an alkali metal ion, an alkali earth metal ion, an ammonium ion and a hydrogen ion; M2 is an ion of a tetravalent metal selected from zirconium and titanium; n is a number satisfying the condition of 0 ≤ n ≤ 6; a and b are positive numbers satisfying a condition of a + mb = 1 or a + mb = 2; and c = 2 and d = 3 when a + mb = 1, and c = 1 and d = 2 when a + mb = 2,
wherein therapeutic uses are excluded.

2. Use according to claim 1, wherein the silver ion carrier has a particle diameter of 0.1 to 15 µm.

3. Use according to claims 1 to 2, wherein the coronavirus is SARS virus.

4. Use according to any one of claims 1 to 3 for the production of a filament.

5. Use according to any one of claims 1 to 3 for the production of a moulded product.

6. Use according to any one of claims 1 to 3 for the production of an UV curing acryl paint.

## Patentansprüche

1. Verwendung eines Mittels, welches einen Silberionenträger umfasst, der aus Verbindungen gemäß der folgenden Formel (1) ausgewählt ist, welcher Silberionen mit einer Konzentration von 0,05 bis 15 Massen-% enthält, zur Inaktivierung eines Coronavirus:
AgₐA_{b}M2_{c}(PO₄)d · nH₂O (1)
wobei A wenigstens ein m-wertiges Ion ist (m ist eine positive ganze Zahl), das aus einem Alkalimetallion, einem Erdalkalimetallion, einem Ammoniumion und einem Wasserstoffion ausgewählt ist; M2 ein Ion eines vierwertigen Metalls ist, das aus Zirconium und Titanium ausgewählt ist; n eine Zahl ist, welche die Bedingung 0 ≤ n ≤ 6 erfüllt; a und b positive Zahlen sind, die eine Bedingung a + mb = 1 oder a + mb = 2 erfüllen; und c = 2 und d = 3 sind, wenn a + mb = 1 ist, und c = 1 und d = 2 sind, wenn a + mb = 2 ist,
wobei therapeutische Verwendungen ausgeschlossen sind.

2. Verwendung gemäß Anspruch 1, wobei der Silberionenträger einen Partikeldurchmesser von 0,1 bis 15 µm aufweist.

3. Verwendung gemäß den Ansprüchen 1 bis 2, wobei das Coronavirus ein SARS-Virus ist.

4. Verwendung gemäß irgendeinem der Ansprüche 1 bis 3 zur Herstellung einer Faser.

5. Verwendung gemäß irgendeinem der Ansprüche 1 bis 3 zur Herstellung eines Formartikels.

6. Verwendung gemäß irgendeinem der Ansprüche 1 bis 3 zur Herstellung einer UV-härtenden Acrylfarbe.

## Revendications

1. Utilisation d'un agent comprenant un support d'ions argent choisi parmi les composés représentés par la formule (1) suivante contenant des ions argent à une concentration de 0,05 à 15 % en poids pour l'inactivation d'un coronavirus :
AgₐA_{b}M2_{c}(PO₄)_{d} · nH₂O (1)
dans laquelle A est au moins un ion de valence m (m est un entier positif) choisi parmi un ion de métal alcalin, un ion de métal alcalino-terreux, un ion ammonium et un ion hydrogène ; M2 est un ion d'un métal tétravalent choisi parmi le zirconium et le titane ; n est un nombre satisfaisant la condition 0 ≤ n ≤ 6 ; a et b sont des nombres positifs satisfaisant une condition a + mb = 1 ou a + mb = 2 ; et c = 2 et d = 3 lorsque a + mb = 1 et c = 1 et d = 2 lorsque a + mb = 2,
dans laquelle des utilisations thérapeutiques sont exclues.

2. Utilisation selon la revendication 1, dans laquelle le support d'ions argent a un diamètre de particule de 0,1 à 15 µm.

3. Utilisation selon la revendication 1 ou 2, dans laquelle le coronavirus est le virus SARS.

4. Utilisation selon l'une quelconque des revendications 1 à 3 pour la production d'un filament.

5. Utilisation selon l'une quelconque des revendications 1 à 3 pour la production d'un produit moulé.

6. Utilisation selon l'une quelconque des revendications 1 à 3 pour la production d'une peinture acrylique durcissable aux UV.
